(19) Europäisches Patentamt
European Patent Office
Office européen des brevets

(11) **EP 1 606 240 B1**

(12) **EUROPÄISCHE PATENTSCHRIFT**

(45) Veröffentlichungstag und Bekanntmachung des Hinweises auf die Patenterteilung:
**06.10.2010   Patentblatt 2010/40**

(21) Anmeldenummer: **04716587.3**

(22) Anmeldetag: **03.03.2004**

(51) Int Cl.:
***C07C 45/83*** *(2006.01)*     ***C07C 47/04*** *(2006.01)*
***C07C 47/058*** *(2006.01)*

(86) Internationale Anmeldenummer:
**PCT/EP2004/002121**

(87) Internationale Veröffentlichungsnummer:
**WO 2004/078690 (16.09.2004 Gazette 2004/38)**

(54) **VERFAHREN ZUR BEREITSTELLUNG HOCHKONZENTRIERTEN GASFÖRMIGEN FORMALDEHYDS**

METHOD FOR PREPARING HIGHLY CONCENTRATED, GASEOUS FORMALDEHYDES

PROCEDE DE PREPARATION DE FORMALDEHYDES SOUS FORME GAZEUSE, HAUTE CONCENTRATION

(84) Benannte Vertragsstaaten:
**AT BE BG CH CY CZ DE DK EE ES FI FR GB GR HU IE IT LI LU MC NL PL PT RO SE SI SK TR**

(30) Priorität: **04.03.2003   DE 10309288**

(43) Veröffentlichungstag der Anmeldung:
**21.12.2005   Patentblatt 2005/51**

(73) Patentinhaber: **BASF SE**
**67056 Ludwigshafen (DE)**

(72) Erfinder:
• **STROEFER, Eckhard**
**68163 Mannheim (DE)**
• **LANG, Neven**
**68163 Mannheim (DE)**
• **STEINBRENNER, Ulrich**
**67435 Neustadt (DE)**
• **HASSE, Hans**
**67661 Kaiserslautern (DE)**
• **OTT, Michael**
**69151 Neckargemünd (DE)**
• **GRÜTZNER, Thomas**
**70567 Stuttgart (DE)**

(56) Entgegenhaltungen:
EP-A- 1 063 221     WO-A-99/21818
FR-A- 2 492 367     GB-A- 1 190 682

EP 1 606 240 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft ein Verfahren zur Herstellung von hochkonzentriertem gasförmigem Formaldehyd.

Formaldehyd ist eine wichtige Industriechemikalie und wird zur Herstellung zahlreicher Industrieprodukte und Verbrauchsartikel eingesetzt. In über 50 Industriezweigen wird derzeit Formaldehyd verwendet, im Wesentlichen in Form von wässrigen Lösungen oder Formaldehyd enthaltenden Kunstharzen. Kommerziell erhältliche, wässrige FormaldehydLösungen weisen Gesamtkonzentrationen von 20 bis 55 Gew.-% Formaldehyd in Form von monomerem Formaldehyd, Methylenglykol und oligomeren Polyoxymethylenglykolen auf. GB 1 190 682 beschreibt die Aufkonzentrierung von wässrigen Formaldehydlösungen.

Wasser, monomerer (freier) Formaldehyd, Methylenglykol und oligomere Polyoxymethylenglykole unterschiedlicher Kettenlänge liegen in wässrigen Lösungen nebeneinander in einem thermodynamischen Gleichgewicht vor, das durch eine bestimmte Verteilung der Polyoxymethylenglykole unterschiedlicher Länge gekennzeichnet ist. Der Begriff "wässrige Formaldehydlösung" bezieht sich dabei auch auf Formaldehydlösungen, die praktisch kein freies Wasser, sondern im Wesentlichen nur noch in Form von Methylenglykol bzw. in den endständigen OH-Gruppen der Polyoxymethylenglykole chemisch gebundenes Wasser enthalten. Dies ist insbesondere bei konzentrierten Formaldehydlösungen der Fall. Polyoxymethylenglykole können dabei beispielsweise zwei bis neun Oxymethyleneinheiten aufweisen. In wässrigen Formaldehydlösungen können also Dioxymethylenglykol, Trioxymethylenglykol, Tetraoxymethylenglykol, Pentaoxymethylenglykol, Hexaoxymethylenglykol, Heptaoxymethylenglykol, Octaoxymethylenglykol und Nonaoxymethylenglykol nebeneinander vorliegen. Die Verteilung ist konzentrationsabhängig. So liegt das Maximum der Verteilung in verdünnten Formaldehydlösungen bei Homologen niedriger Kettenlänge, während es in konzentrierteren Formaldehydlösungen bei Homologen höherer Kettenlänge liegt. Eine Gleichgewichtsverschiebung hin zu längerkettigen (höhermolekularen) Polyoxymethylenglykolen kann durch Wasserentzug, beispielsweise durch einfache Destillation in einem Filmverdampfer, erfolgen. Die Gleichgewichtseinstellung erfolgt dabei mit endlicher Geschwindigkeit durch die intermolekulare Kondensation von Methylenglykol und niedermolekularen Polyoxymethylenglykolen unter Wasserabspaltung zu höhermolekularen Polyoxymethylenglykolen.

Der Einsatz von gasförmigem Formaldehyd ist in vielen Fällen vorteilhaft. Gasförmig liegt Formaldehyd überwiegend in seiner hochreaktiven monomeren Form vor. Gasförmige Mischungen, die weitgehend frei von Wasser und Hydroxyverbindungen sind, können in Umsetzungen eingesetzt werden, wo letztere störend sind, da sie beispielsweise zu Nebenreaktionen führen oder die Selektivität vermindern oder auch den Katalysator desaktivieren - beispielsweise bei der Katalyse mit Lewis-Säuren. Häufig ist es auch von Vorteil, Umsetzungen als Mehrphasenreaktionen unter Beteiligung einer Gasphase durchzuführen. Es besteht somit ein Bedarf an Verfahren zur Bereitstellung von gasförmigen Formaldehyd/Wasser-Gemischen mit einem hohen Formaldehyd/$H_2$O-Verhältnis.

Gasförmiger Formaldehyd kann im Labormaßstab durch Erhitzen von Paraformaldehyd hergestellt werden. Der Umgang mit Paraformaldehyd erfordert jedoch aufwendige Einrichtungen zur Handhabung von Feststoffen. Paraformaldehyd wird daher im großtechnischen Maßstab nicht genutzt.

Bekannt ist ferner die Herstellung von gasförmigem Formaldehyd durch Gewinnung von Halbacetalen des Formaldehyds und nachfolgender Spaltung der Halbacetale in Alkohol und freien Formaldehyd. Ein entsprechendes Verfahren ist z.B. in DE-A 41 37 846 beschrieben. Es erfordert eine Extraktionsstufe, in der der Formaldehyd aus einer wässrigen Formalin-Lösung als Halbacetal extrahiert wird. Das gewonnene Halbacetal wird getrocknet und schließlich thermisch in Formaldehyd und den Alkohol gespalten, wobei der Alkohol abgetrennt und in die Extraktionsstufe zurückgeführt wird. Dieses Verfahren ist aufwendig. Ferner wird der Alkohol als weiterer Stoff in das Verfahren eingeführt. Dies ist teuer und kann zu Verunreinigungen des Formaldehyds mit dem Alkohol führen.

Durch heterogenkatalytische, oxidative Dehydrierung von Methan wird, wie in DE-A 199 10 145 beschrieben, ebenfalls ein Formaldehyd/Wasser-Gemisch als Reaktionsaustrag erhalten. Da zur Vermeidung der Katalysatordesaktivierung große Mengen Frischwasser dem Einsatzgasstrom zugegeben werden müssen, löst dieses Verfahren das Problem, Gasgemische mit hohem Formaldehyd/Wasser-Verhältnis bereitzustellen, nicht.

Die nicht oxidative Dehydrierung von Methanol unter Bildung von Formaldehyd und Wasserstoff ist in neuerer Zeit intensiv untersucht worden und ist beispielsweise in DE-A 198 10 087 und DE-A 198 22 598 beschrieben. Dabei wird die klassische Route der oxidativen Dehydrierung von Methanol unter Bildung wässriger Formaldehydlösungen verlassen. Dampfförmiges Methanol wird an einem aerosolischen Katalysator dehydriert. Die Reaktion ist endotherm und muss aufgrund ihrer thermodynamischen Limitierung bei hohen Temperaturen durchgeführt werden, um einen hohen Umsatz zu erzielen. Ein hoher bis vollständiger Umsatz des Methanols ist jedoch notwendig, damit das Verfahren wirtschaftlich ist, und um die Bildung von Halbacetalen und Acetalen aus Formaldehyd und nicht umgesetztem Methanol zu verhindern. Die hohen Betriebstemperaturen erfordern aber hohe Investitionen und Energiekosten.

Der im Acetal gebundene Alkohol kann die Folgereaktionen des Formaldehyds stören. Auch ist die Abtrennung des aerosolischen Katalysators aus dem Produktgas aufwendig. Diese Abtrennung muss aber möglichst vollständig sein, wenn der Aerosol-Katalysator die Katalysatoren desaktiviert, die die Folgereaktionen des Formaldehyds katalysieren. Schließlich zerfällt ein Teil des Methanols bei den hohen Temperaturen der nicht oxidativen Methanol-Dehydrierung in CO und Wasserstoff.

Es empfiehlt sich daher, beim klassischen Verfahren der oxidativen Methanol-Dehydrierung zu bleiben. Dieses Verfahren hat sich bewährt, ist technisch ausgereift und immer weiter verbessert worden. Dabei wird Formaldehyd mit einer Selektivität von > 90% gebildet.

Aus der bei der klassischen oxidativen Methanol-Dehydrierung anfallenden wässrigen Formaldehydlösung kann gasförmiger Formaldehyd gewonnen werden. Während die flüssige Phase die oben beschriebene Verteilung von Polyoxymethylenglykolen unterschiedlicher Kettenlänge aufweist, liegt Formaldehyd in der Gasphase als Methylenglykol $CH_2(OH)_2$ und freier Formaldehyd $CH_2O$ vor. Wird beispielsweise eine 30 gew.-%ige wässrige Formaldehydlösung auf eine Temperatur von 107°C oder darüber erwärmt, so kann ein gasförmiges Formaldehyd/Wasser-Gemisch abgezogen werden, dessen Formaldehyd-Gehalt, berechnet als $CH_2O$, bei 25 bis 35 Gew.-% liegt. Daraus ergibt sich ein molares Verhältnis von $CH_2O$ zu Wasser ($CH_2O : H_2O$) von 0,2 bis 0,32.

Die Verdampfung von wässriger Formaldehydlösung führt jedoch bei hochkonzentrierten Formaldehydlösungen zum Ausfall von Feststoffen. Legt man beispielsweise eine Formaldehydlösung mit einem $CH_2O$-Gehalt von 70 Gew.-% in einem Rundkolben vor und versucht, bei Umgebungsdruck zu verdampfen, so kommt es nach dem Abdampfen einer bestimmten Flüssigkeitsmenge zu Feststoffausfall.

Aufgabe der Erfindung ist es, ein wirtschaftliches Verfahren zur Herstellung von hochkonzentriertem gasförmigem Formaldehyd mit einem molaren $CH_2 : H_2O$ - Verhältnis von $\geq 0,6$ bereit zu stellen.

Gelöst wird die Aufgabe durch ein Verfahren zur Herstellung von hochkonzentriertem gasförmigem Formaldehyd mit einem molaren $CH_2O : H_2O$ -Verhältnis von $\geq 0,6$ aus einer wässrigen Formaldehydlösung durch Verdampfen zumindest eines Teils dieser Lösung, bei dem die wässrige Formaldehydlösung auf eine Verdampfungstemperatur T erwärmt wird und die gebildete Gasphase abgezogen wird, wobei für die Verdampfungstemperatur T gilt:

$$T \, [°C] \geq T'_{min} \, [°C]$$

mit $T'_{min}(c) = A + B \times (c/100) + C \times (c/100)^2 + D \times (c/100)^3$ und
A = + 68,759, B = + 124,77, C = -12,851, D = - 10,095, wobei c der aktuelle $CH_2O$-Gehalt der wässrigen Formaldehydlösung während der Verdampfung in Gew.-% ist und von 20 bis 99 Gew.-% beträgt.

Bevorzugt wird von einer wässrigen Formaldehydlösung mit einem $CH_2O$-Gehalt von 50 bis 99 Gew.-%, besonders bevorzugt von 70 bis 95 Gew.-%, insbesondere von 70 bis 90 Gew.-% ausgegangen.

Der $CH_2O$-Gehalt der wässrigen Formaldehydlösung ist der Gehalt an Formaldehyd in freier, monomerer Form, in Form von Methylenglykol oder Polyoxymethylenglykolen, berechnet als $CH_2O$.

Die Einstellung der Verdampfungstemperatur erfolgt durch Wahl des Drucks während des Verdampfungsvorganges. Dieser wird so gewählt, dass sich die resultierende Verdampfungstemperatur T(p) während des gesamten Verdampfungsvorganges, bei dem die wässrige Formaldehyd-Ausgangslösung teilweise oder vollständig verdampft und so der hochkonzentrierte gasförmige Formaldehyd erhalten wird, oberhalb des durch die obige Formel definierten Grenzwertes bewegt. Dieser Grenzwert ist konzentrationsabhängig, wobei T(c) die Abhängigkeit dieses Grenzwertes von der aktuellen Formaldehydkonzentration der wässrigen Formaldehydlösung zu jedem Zeitpunkt des Verdampfungsvorganges angibt. Wie obiger Formel zu entnehmen ist, steigt der Temperaturgrenzwert mit der Formaldehydkonzentration an. So kann es notwendig sein, bei sukzessiver Aufkonzentrierung der wässrigen Formaldehydlösung den Druck zu erhöhen, um den Temperaturgrenzwert nicht zu unterschreiten.

Im Allgemeinen liegt der Druck während der partiellen Verdampfung im Bereich von 0,1 bis 50 bar, bevorzugt im Bereich von 0,5 bis 17 bar.

Die Verdampfung der wässrigen Formaldehydlösung kann teilweise oder vollständig, kontinuierlich oder diskontinuierlich erfolgen.

Erfolgt die Verdampfung vollständig, so weist die erhaltene Gasphase insgesamt den gleichen $CH_2O$-Gehalt wie die zu Beginn der Verdampfung vorhandene flüssige Phase auf. Überraschender Weise liegt auch bei nur teilweiser Verdampfung die Formaldehyd-Konzentration - berechnet als $CH_2O$ - in der Gasphase nahe an oder oberhalb der Formaldehyd-Konzentration - berechnet als $CH_2O$ - in der Flüssigphase. Damit verhält sich das System über einen weiten Konzentrationsbreich ähnlich einem Azeotrop. Dies kann möglicher Weise darauf zurückzuführen sein, dass durch Depolymerisation der in der Lösung vorhandenen Polyoxymethylenglykole während des Verdampfungsvorgangs kontinuierlich monomerer Formaldehyd nachgebildet wird, während durch Kondensation von kürzerkettigen zu längerkettigen Polyoxymethylenglykolen kontinuierlich Wasser freigesetzt wird.

Somit werden gasförmige Formaldehyd/Wasser-Gemische erhalten, in denen das molare Verhältnis von Formaldehyd zu Wasser ($CH_2O : H_2O$) $\geq 0,6$ ist. Bevorzugt ist dieses Verhältnis $\geq 1,4$, besonders bevorzugt $\geq 1,6$.

Die Verdampfung der wässrigen Formaldehydlösung kann in Gegenwart saurer oder basischer Katalysatoren durchgeführt werden, welche die oben skizzierten Depolymerisations- und Kondensationsreaktionen katalysieren. Jedoch wird man aus Kostengründen, und auch um Ablagerungen an den Wärmetauscherflächen zu vermeiden, die Katalysatorzusätze gering halten. Die Katalyse kann homogen oder auch heterogen in Suspensions- oder Festbettfahrweise erfolgen.

Die Verdampfung kann in handelsüblichen Apparaten erfolgen. Geeignet sind beispielsweise Rührkessel, die z.B. durch Doppelmäntel oder Rohrschlangen (innenliegend oder außen angebracht) geheizt werden können. Besonders geeignet sind Apparate mit Wärmetauschercharakteristik, z.B. Rohrbündelwärmetauscher, Plattenapparate oder Wendelrohre. Diese können im Gleichstrom, Gegenstrom- oder Kreuzstrom betrieben werden. Die Beheizung kann durch beliebige Medien erfolgen, beispielsweise mit kondensierendem Dampf oder einphasig durch Flüssigkeiten oder Gase. Die Verdampfung der wässrigen Formaldehydlösung kann in einmaligem Durchgang durch den Verdampfer oder im Umlauf erfolgen. Insbesondere dann, wenn vollständige Verdampfung angestrebt wird, wird ein einmaliger Durchlauf der Formaldehydlösung durch den Verdampfer im Allgemeinen nicht ausreichen.

Die Formaldehydlösung wird bei einer Temperatur verdampft, bei der kein Feststoff ausfällt. Bevorzugt ist es, an jeder Stelle in dem Verdampfer eine Temperatur aufrecht zu erhalten, bei der kein Feststoff ausfällt. Beispielsweise wird diese Temperatur sowohl in dem Verdampfer selbst als auch - bei Betrieb des Verdampfers im Umlauf - in dem Umlauf und bei Entnahme der wässrigen Formaldehydlösung in den dem Verdampfer nachgeschalteten Einrichtungen aufrechterhalten.

Dazu wird an jeder Stelle in dem Verdampfer eine Temperatur

$$T \geq T''_{min}$$

mit $T''_{min}(c) = A' + B' \times (c/100) + C' \times (c/100)^2 + D' \times (c/100)^3$
und
$A' = + 6,0156$, $B' = + 52,918$, $C' = + 49,699$, $D' = + 34,286$.
wobei c der aktuelle $CH_2O$-Gehalt der wässrigen Formaldehydlösung während der Verdampfung in Gew.-% ist und 20 bis 99 Gew.-% beträgt,
in der wässrigen Formaldehydlösung aufrechterhalten.

Die Verdampfungstemperatur sollte allerdings nicht einen oberen Temperaturgrenzwert $T_{max}$ übersteigen, da bei zu hohen Temperaturen Zersetzung des gasförmigen Formaldehyds in CO und Wasserstoff stattfindet. Dieser obere Temperaturgrenzwert beträgt im Allgemeinen 300°C, bevorzugt 200°C.

Die Herstellung des hochkonzentrierten gasförmigen Formaldehyds kann auch in einem Filmverdampfer oder Dünnschichtverdampfer erfolgen. Die Herstellung kann auch in einem Wendelrohrverdampfer erfolgen, wie er in DE-A 27 19 967 beschrieben ist. Ein geeigneter Filmverdampfer ist in der Figur gezeigt. Es handelt sich hierbei um einen Dünnschichtverdampfer. Der Zulauf 1, bestehend aus Rohlösung (Ausgangsstoffgemisch) und gegebenenfalls Rückführstrom, wird zunächst einem Flüssigkeitsverteiler 2 zugeführt. Dieser verteilt die Rohlösung auf eine Verdampferfläche 3. Die Verdampferfläche 3 (Wärmetauscherfläche) ist üblicherweise zylindrisch geformt, kann jedoch auch zumindest teilweise konische Form aufweisen. Sie steht mit der Innenseite eines Heizmantels 4, der für eine Wärmezufuhr zur Verdampferfläche 3 sorgt, in thermischem Kontakt. Der Flüssigkeitsverteiler 2 trägt dazu bei, dass die Zulauflösung gleichmäßig auf den Umfang der Verdampferfläche 3 verteilt wird.

Rotierende Wischerblätter 5 verteilen die Lösung sodann weiter über die Verdampferfläche 3, sorgen für eine Aufrechterhaltung und Förderung eines Flüssigkeitsfilmes auf der Verdampferfläche 3 und tragen zur Intensivierung des Wärme- und Stofftransportes in der Flüssigkeit bei. Diese Wischerblätter 5 werden von einer Antriebsvorrichtung 6 angetrieben. Je nach Gestaltung und Positionierung der Wischerblätter 5 kann dabei der Flüssigkeitsfilm eher dünn gehalten oder aufgestaut werden. Damit ist eine Veränderung der Verweilzeit bzw. der Verweilzeitverteilung der Lösung im Filmverdampfer möglich. Die typische Verweilzeit der Lösung im Filmverdampfer beträgt zwischen 1 s und 10 min, bevorzugt zwischen 2 s und 2 min.

Durch einen Heizmittelzulauf 7 wird ein Heizmittel, z.B. Wasserdampf, in den Heizmantel geführt. Dieses heizt die Verdampferfläche auf. Abgekühltes Heizmittel, z.B. kondensiertes Wasser im Falle von Wasserdampf als Heizmittel, wird über den Heizmittelablauf 8 abgeführt.

Durch die Wärmezufuhr zur Verdampferfläche 3 wird ein Teil der dem Filmverdampfer zugeführten Lösung verdampft.

Der entstandene Brüden (d.h. Dampf bzw. Gase) gelangt in einen Phasentrennraum 9 und von dort in einen Tropfenabscheider 10. Mit dem Brüden mitgerissene Flüssigkeitströpfchen werden hier aus der Gasphase entfernt und in die Flüssigkeit (Lösung) zurückgeführt. Das Konzentrat 13 wird auf geeignete Weise aus dem Phasentrennraum 9 ausgeleitet, während der Brüden 12 aus dem Tropfenabscheider 10 abgezogen wird.

Wenn in dem beschriebenen Filmverdampfer eine wässrige Formaldehydlösung eingeleitet wird, reichern sich, bei einer Temperatur $T < T'_{min}$, in der Flüssigkeit 13 die Polyoxymethylenglykole an, während das Kondensat aus dem Brüden 12 arm an Polyoxymethylenglykolen und reich an Formaldehyd, Methylenglykol und Wasser ist. Liegt die Temperatur aber oberhalb des durch die obige Formel definierten Grenzwertes $T'_{min}$, so weist die Gasphase einen Formaldehyd-Gehalt auf, der dem Formaldehyd-Gehalt der flüssigen Phase sehr nahe kommt.

Der Kondensator kann in einer besonderen Ausführungsform in den Verdampfungskörper integriert sein, wodurch sich eine kürzere Verweilzeit der verdampften Komponenten in der Dampfphase sowie eine kompaktere Bauweise ergeben.

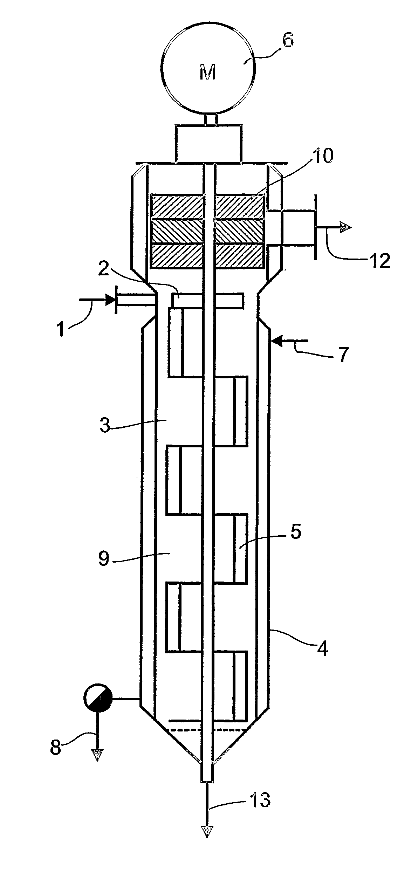

Neben der in Fig. 1 dargestellten Ausführung eines Filmverdampfers kann auch ein Apparat ohne mechanische Beeinflussung des Flüssigkeitsfilmes auf der Verdampfungsfläche eingesetzt werden. Die Wärmeübertragungsfläche dieser Fallfilm- oder Fallstromverdampfer kann dabei als Rohre oder Platten ausgebildet sein.

Das erfindungsgemäße Verfahren geht vorzugsweise von mäßig oder hochkonzentrierten Formaldehydlösungen mit einem CH$_2$O-Gehalt von 50 bis 99 Gew.-% aus, die nach ihrer Herstellung wie nachfolgend beschrieben gegen Feststoffausfall stabilisiert wurden.

Höher konzentrierte Formaldehydlösungen mit beispielsweise > 70 Gew.-% CH$_2$O fallen bei der Herstellung bei niedrigen Temperaturen von ca. 20 bis 50°C zunächst einphasig an. Nach einer gewissen Zeit kommt es jedoch zum Feststoffausfall. Ursache scheint das Anwachsen der Polyoxymethylenglykol-Ketten in der Formaldehydlösung bis zur Überschreitung der Löslichkeitsgrenze zu sein. Diese Lösungen können gegen Feststoffausfall stabilisiert werden, indem sie unmittelbar nach der Herstellung mit einer Aufheizrate von mindestens 5°C/min auf eine Temperatur von mindestens 80°C bis höchstens 200°C erwärmt und bei einer Temperatur in diesem Bereich belassen werden. Unmittelbar nach der Herstellung bedeutet, dass die beispielsweise bei 20 bis 60°C erhaltene hochkonzentrierte Formaldehydlösung nach spätestens 60 min, bevorzugt nach spätestens 5 min, mit der spezifizierten Aufheizrate erwärmt wird.

Vorzugsweise beträgt die Aufheizrate mindestens 10°C/min. Eine Aufheizrate von mindestens 10°C/min ist insbesondere dann bevorzugt, wenn der pH-Wert der Lösung < 3 oder > 6 beträgt. Vorzugsweise wird mit der spezifizierten Aufheizrate auf mindestens 100°C erwärmt und diese Temperatur anschließend nicht mehr unterschritten. Der pH-Wert der hochkonzentrierten Formaldehydlösung liegt üblicherweise im Bereich von 1 bis 10, vorzugsweise 2 bis 9, besonders bevorzugt von 6 bis 8. Der pH-Wert kann durch Zugabe von Puffersubstanzen, beispielsweise einem Formiat-Puffer, auf den gewünschten Bereich eingestellt werden.

Vorzugsweise werden Stabilisierung und Verdampfung der hochkonzentrierten wässrigen Formaldehydlösungen in einem Apparat bei erhöhtem Druck durchgeführt.

Der erhaltene hochkonzentrierte gasförmige Formaldehyd kann für eine Vielzahl chemischer Umsetzungen verwendet werden. Beispiele für derartige Umsetzungen sind

• die Umsetzung von Acetylen mit Formaldehydlösung in einer Reppe-Reaktion zu Butindiol, das zu Butandiol weiter hydriert werden kann;

• Aldolisierungsreaktionen von Formaldehyd mit sich selbst oder höheren Aldehyden zu mehrwertigen Alkoholen und Zuckern, Pentaerythrit, Trimethylolpropan und Neopentylglykol;

• die Umsetzung von Formaldehyd und CO zu Glykolsäure;

• die Herstellung chelatisierender Substanzen wie Glykolnitrile aus Lösungen von Formaldehyd;

• die Umsetzung von Formaldehyd mit Olefinen in einer Prins-Reaktion zu alpha-Hydroxymethylverbindungen;

• Kondensationsreaktionen von Formaldehyd mit Aminen, wie Anilin oder Toluidin, zu Schiffschen Basen, die zu Diphenylmethanderivate, wie Methandiphenyldiamin weiterreagieren können;

• Umsetzung von Hydroxylamin mit Formaldehyd zu Oximen;

• Umsetzung von Formaldehyd mit Diolen zu cyclischen Ethern, beispielsweise von Glykol und Formaldehyd zu Dioxolan.

In einer besonders bevorzugten Verwendung wird das gasförmige Formaldehyd/WasserGemisch einer Trioxan- oder Tetraoxan-Synthese zugeführt. Dabei kann das gasförmige Gemisch mit dem Fachmann bekannten Methoden entwässert werden. Die Trioxan-Synthese ist beispielsweise in AT 252913 beschrieben.

Die Listung ist nicht vollständig. Lehrbücher der organischen Chemie und der technischen Chemie enthalten weitere Beispielreaktionen. Die Listung soll aber beispielhaft die industrielle Bedeutung des Formaldehyds als Synthesebaustein im gesamten Bereich der organischen Chemie verdeutlichen. Dies betrifft sowohl kleintonnagige Zwischenprodukte im Pharma- oder Pflanzenschutzbereich wie z.B. Oxime als auch großtonnagige Produkte wie Diphenylmethanderivate.

Die Erfindung wird durch das nachstehende Beispiel näher erläutert.

**Beispiel**

In einem Laborversuch wird in einem Dünnschichtverdampfer gemäß Abb. 1 im geraden Durchgang eine hochkonzentrierte Formaldehydlösung hergestellt. Der Verdampfer weist eine Verdampffläche von 0,092 m$^2$ und eine Länge von 1,1 m auf. Am Kopf wird eine 48 gew.-%ige wässrige Formalinlösung mit einem Mengenstrom von 615 g/h zugegeben. Die Wandtemperatur beträgt 90°C, der Druck beträgt 80 mbar. Am Sumpf werden 298 g/h einer 84 gew.-%igen hochkonzentrierten Formaldehydlösung abgezogen. Am Kopf werden 321 g/h Brüden abgezogen.

Die Sumpflösung wird mit einer Laborpumpe in einen geheizten Standard-Laborrührreaktor von 1 Inhalt gefördert. Der Reaktor wird auf einer Innentemperatur von 155-160°C gehalten, wobei sich ein Druck von etwa 8 bar einstellt. Aus dem Reaktor wird ein Füssigkeitsstrom von 27 g/h entnommen. Aus der Gasphase des Reaktors wird ein Gasstrom von 268 g/h abgezogen. Dieser enthält nach Analyse 82 bis 85 Gew.-%, berechnet als $CH_2O$.

## Patentansprüche

1. Verfahren zur Herstellung von hochkonzentriertem gasförmigem Formaldehyd mit einem molaren $CH_2O : H_2O$ -Verhältnis von ≥ 0,6 aus einer wässrigen Formaldehydlösung durch Verdampfen zumindest eines Teils dieser Lösung, bei dem die wässrige Formaldehydlösung auf eine Verdampfungstemperatur T erwärmt wird und die gebildete Gasphase abgezogen wird, wobei für die Verdampfungstemperatur T gilt:

$$T \ [°C] \geq T'_{min} \ [°C]$$

mit $T'_{min}(c) = A + B \times (c/100) + C \times (c/100)^2 + D \times (c/100)^3$
und
A = + 68,759, B = + 124,77, C = - 12,851, D = - 10,095, wobei c der aktuelle $CH_2O$-Gehalt der wässrigen Formaldehydlösung während der Verdampfung in Gew.-% ist und von 50 bis 99 Gew.-% beträgt.

2. Verfahren nach Anspruch 1, **dadurch gekennzeichnet, dass** die wässrige Formaldehydlösung einen $CH_2O$-Gehalt von 70 bis 90 Gew.-% aufweist.

3. Verfahren nach einem der Ansprüche 1 oder 2, **dadurch gekennzeichnet, dass** der Druck während der Verdampfung von 0,1 bis 50 bar beträgt.

4. Verfahren nach einem der Ansprüche 1 bis 3, **dadurch gekennzeichnet, dass** das molare $CH_2O$: $H_2O$ -Verhältnis ≥ 1,4 ist.

5. Verfahren nach einem der Ansprüche 1 bis 4, **dadurch gekennzeichnet, dass** an jeder Stelle in dem Verdampfer eine Temperatur

$$T \ [°C] \geq T''_{min} \ [°C]$$

mit $T^2_{min}(C) = A' + B' \times (c/100) + C' \times (c/100)^2 + D' \times (c/100)^3$
und

A' = + 6,0156, B' = + 52,918, C' = + 49,699, D' = + 34,286.
wobei c der aktuelle $CH_2O$-Gehalt der wässrigen Formaldehydlösung während der Verdampfung in Gew.-% ist und von 50 bis 99 Gew.-% beträgt, in der wässrigen Formaldehydlösung aufrechterhalten wird.

6. Verfahren nach einem der Ansprüche 1 bis 5, **dadurch gekennzeichnet, dass** die Verdampfung in einem Rührkessel, Wendelrohr, Filmverdampfer oder einem anderen Apparat mit Wärmetauschercharakteristik erfolgt.

7. Verfahren nach einem der Ansprüche 1 bis 6, **dadurch gekennzeichnet, dass** die wässrige Formaldehydlösung, von der das Verfahren ausgeht, durch oxidative Dehydrierung von Methanol hergestellt wird.

## Claims

1. A process for preparing high-concentration gaseous formaldehyde having a $CH_2O: H_2O$ molar ratio of ≥ 0.6 from an aqueous formaldehyde solution by evaporation of at least part of this solution, in which the aqueous formaldehyde solution is heated to an evaporation temperature T and the gas phase formed is taken off, wherein the evaporation temperature T obeys the relationship:

$$T \ [°C] \ \geq T'min \ [°C]$$

where $T'min (c) = A + B \times (c/100) + C \times (c/100)^2 + D \times (c/100)^3$
and
A = + 68.759, B = + 124.77, C = - 12.851, D = - 10.095, where c is the instantaneous $CH_2O$ content of the aqueous formaldehyde solution during the evaporation in percent by weight and is from 50 to 99% by weight.

2. The process according to claim 1, wherein the aqueous formaldehyde solution has a $CH_2O$ content of from 70 to 90% by weight.

3. The process according to either of claims 1 and 2, wherein the pressure during the evaporation is from 0.1 to 50 bar.

4. The process according to any of claims 1 to 3, wherein the $CH_2O$: $H_2O$ molar ratio is ≥ 1.4.

5. The process according to any of claims 1 to 4, wherein a temperature which obeys the relationship

$$T\ [°C] \geq T''_{min}\ [°C]$$

where $T^2_{min}(c) = A' + B' \times (c/100) + C' \times (c/100)^2 + D' \times (c/100)^3$
and
A' = + 6.0156, B' = + 52.918, C' = + 49.699, D' = + 34.286,
where c is the instantaneous $H_2O$ content of the aqueous formaldehyde solution during the evaporation in percent by weight and is from 50 to 99% by weight, is maintained in the aqueous formaldehyde solution at every point in the evaporator.

6. The process according to any of claims 1 to 5, wherein the evaporation takes place in a stirred vessel, helically wound tube, film evaporator or another apparatus having heat exchanger characteristics.

7. The process according to any of claims 1 to 6, wherein the aqueous formaldehyde solution used as starting material for the process is prepared by oxidative dehydrogenation of methanol.

**Revendications**

1. Procédé pour la fabrication de formaldéhyde gazeux fortement concentré, ayant une proportion molaire en $CH_2O : H_2O \geq 0,6$, à partir d'une solution aqueuse de formaldéhyde, par évaporation d'au moins une partie de cette solution, dans lequel on chauffe la solution aqueuse de formaldéhyde à une température d'évaporation T, et on extrait la phase gazeuse formée, la température d'évaporation T répondant à la relation suivante :

$$T\ [°C] \geq T'_{min}\ [°C]$$

avec $T'_{min}(c) = A + B \times (c/100) + C \times (c/100)^2 + D \times (c/100)^3$
et
A = + 68,759, B = + 124,77, C = - 12,851, D = - 10,095,
la valeur c représentant la teneur en $CH_2O$ présente dans la solution aqueuse de formaldéhyde au cours de l'évaporation, exprimée en % en poids, et dont la valeur est de 50 à 99 % en poids.

2. Procédé selon la revendication 1, **caractérisé en ce que** la solution aqueuse de formaldéhyde présente une teneur en $CH_2O$ de 70 à 90 % en poids.

3. Procédé selon l'une quelconque des revendications 1 ou 2, **caractérisé en ce que** la pression exercée au cours de l'évaporation est de 0,1 à 50 bars.

4. Procédé selon l'une quelconque des revendications 1 à 3, **caractérisé en ce que** la proportion molaire en $CH_2O : H_2O$ est ≥ 1, 4.

5. Procédé selon l'une quelconque des revendications 1 à 4, **caractérisé en ce que** la solution aqueuse de formaldéhyde est maintenue, quel que soit l'endroit dans l'évaporateur, à une température constante répondant à la relation suivante :

$$T\ [°C] \geq T''_{min}\ [°C]$$

avec $T^2_{min}(c)\ A' + B' \times (c/100) + C' \times (c/100)^2 + D' \times (c/100)^3$
et
A' = + 6,0156, B' = + 52,918, C' = + 49,699, D' = + 34,286,
la valeur c représentant la teneur en $CH_2O$ présente dans la solution aqueuse de formaldéhyde au cours de l'évaporation, exprimée en % en poids, et dont la valeur est de 50 à 99 % en poids.

6. Procédé selon l'une quelconque des revendications 1 à 5, **caractérisé en ce que** l'évaporation se fait dans une cuve agitée, un tube spiralé, un évaporateur à film ou un autre appareil doté d'une caractéristique d'échangeur de chaleur.

7. Procédé selon l'une quelconque des revendications 1 à 6, **caractérisé en ce que** la solution aqueuse de formaldéhyde, avec laquelle on démarre le procédé, est obtenue par déshydrogénation oxydative du méthanol.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- GB 1190682 A
- DE 4137846 A
- DE 19910145 A
- DE 19810087 A
- DE 19822598 A
- DE 2719967 A
- AT 252913